# EUROPEAN PATENT APPLICATION

(11) **EP 4 516 802 A1**
(43) Date of publication of application: **05.03.2025**
(21) Application number: 23796840.9
(22) Date of filing: 27.04.2023
(51) Int. Cl.: C07K 7/06, A61P 29/00, A61K 38/00, A61K 8/64, A61Q 19/00

(54) **NOVEL ANTI-INFLAMMATORY PEPTIDE AND USE THEREOF**

(30) Priority: 27.04.2022 KR 20220052169
(71) Applicant: Novacell Technology Inc., Pohang-si, Gyeongsangbuk-do 37673 (KR)
(72) Inventor: LEE, Taehoon, Seoul 06253 (KR); GHIM, Jaewang, Pohang-si Gyeongsangbuk-do 37666 (KR); KIM, Young-Eun, Pohang-si Gyeongsangbuk-do 37666 (KR); JEONG, Min Gyu, Pohang-si Gyeongsangbuk-do 37666 (KR); CHANG, Yeonho, Pohang-si Gyeongsangbuk-do 37666 (KR)
(74) Representative: Vossius & Partner Patentanwälte Rechtsanwälte mbB
(86) International application number: PCT/KR2023/005779
(87) International publication number: WO 2023/211198

(57) **Abstract**

The present invention relates to a novel immunoregulatory peptide having the ability to control inflammatory responses, and a use thereof. The peptide of the present invention may be useful in the treatment of inflammation, various immune diseases, or cancer, by regulating the activity of immune cells.

## Description

### [Technical Field]

The present invention relates to a novel peptide, and a pharmaceutical composition for preventing or treating an immune disease or cancer and an anti-inflammatory cosmetic composition including the same.

### [Background Art]

The resolution of inflammation is a biological mechanism that terminates an innate immune response, restores tissue homeostasis, and induces an appropriate adaptive immune response. In the past, the resolution of inflammation was understood as a passive phenomenon occurring after an inflammatory response, whereas recently, mediators for the resolution of inflammation have been identified, revealing that the resolution of inflammation is an active biological mechanism that progresses step by step according to inflammatory responses involving complex factors. When a wound, contact with a foreign substance, or infection occurs, a defensive mechanism occurs through an acute inflammatory response of the innate immune system, and then the resolution of inflammation action controls the inflammatory response to an appropriate level and induces healing and recovery of damaged tissue. The resolution of inflammation limits further neutrophil infiltration into inflammatory tissue, induces cell death of neutrophil, and mediates decreases in lipid inflammatory substances, cytokines and chemokines, which cause inflammatory responses. Afterward, by inducing the efferocytosis of macrophages, the removal of damaged tissue or dead cells is promoted. To this end, inflammatory macrophages are reduced and an increase in macrophages having anti-inflammatory properties is induced. The appropriate resolution of inflammation mediates a normal adaptive immune response and strengthens the ability to respond to repeated inflammatory situations in the future. However, an insufficient resolution of inflammation occurs excessively/continuously, causing various chronic inflammatory diseases, and it is an abnormal adaptive immune response, causing an autoimmune disease.

The resolution of inflammation is induced by cell-secreted substances, such as pro-resolving factors, called specialized pro-resolving mediators (SPMs). When the resolution of inflammation begins, various types of pro-resolving factors such as lipids, peptides, and proteins control inflammatory responses, such as the activation of immune cells, through specific receptors such as GPCRs.

Current inflammatory treatments inhibit occurrence of inflammatory responses by inhibiting the activity of a specific enzyme or antagonizing a specific receptor or its ligand. Existing anti-inflammatory strategies can effectively and strongly inhibit acute inflammatory responses, but also inhibit normal inflammatory responses, causing various side effects. Concerns rising particularly with patients with chronic inflammation or autoimmune diseases who require long-term drug administration, where the decreased immunity and the risk of secondary infection are the common side effects. Therefore, there is an emerging need for development of novel therapeutic option with an innovative approach to inflammatory response with efficacy on safe inflammatory response control. The strategy of inducing the resolution of inflammation is an innovative immunotherapy strategy that controls excessive/sustained inflammatory responses using an agonist for strengthening the inflammation resolution mechanism. Particularly, it is attracting attention as an alternative to broad-spectrum immunosuppressants, which have a high risk of side effects in the treatment of chronic inflammatory/autoimmune diseases requiring long-term drug administration, due to its limited effect on normal immune responses. Targets of therapeutic strategy for inducing the resolution of inflammation include not only inflammatory diseases that occur due to immune dysfunction in various tissues such as the skin, the respiratory system, the digestive system, the eyes, the musculoskeletal system, and the circulatory system, but also diseases in which an inflammatory response occurring upon onset has a significant impact on the progression of the disease. Particularly Parkinson's disease and Alzheimer's disease, which are well-known neurological diseases in which neuroinflammation greatly affects the worsening of symptoms. In addition, as the importance of the impact of an inflammatory response in the tumor microenvironment on the progression of cancer has been confirmed, especially in solid cancer, anticancer treatment through immunomodulation by induing the resolution of inflammation is also being evaluated as a new strategy.

Formyl-peptide receptor (FPR)-based receptors are G protein-coupled receptors (GPCRs) that are mainly present in immune cells and mediate various cell functions through downstream signaling systems. FPR1 mainly binds to a bacteria-derived peptide to induce an acute inflammatory response and mediates a defensive function, whereas FPR2 has been shown to control inflammatory responses by binding to various types of endogenous ligands (peptides, proteins, and lipids), and is attracting attention as a target for the treatment of inflammatory diseases. FPR2 is a receptor for representative pro-resolving factors (LXA4, AnxA1, Ac2-26, RvD1, etc.) that are expressed in innate immune cells and mediate the induction of the resolution of inflammation, and based on the characteristics of FPR2 with complex ligands, there are attempts to develop various types of substances such as peptides, lipids, proteins, and small molecule compounds as materials for FPR2-targeting treatments for inducing the resolution of inflammation. Such attempts mainly focus on developing treatments based on the research results on the effectiveness in treating various inflammatory diseases such as atopic dermatitis, psoriasis, asthma, lung damage, lung inflammation, conjunctivitis, keratitis, dry eye syndrome, rheumatoid arthritis, ankylosing spondylitis, ulcerative colitis, Crohn's disease, and systemic sclerosis as inflammatory diseases that occur in various tissues such as the skin, the respiratory system, the digestive system, the eyes, the musculoskeletal system, and the circulatory system due to abnormal local or systemic immune responses. In addition, as the importance of neuroinflammation occurring in microglial cells or neurons, participating in immune function in the brain in degenerative brain diseases such as Parkinson's disease and Alzheimer's disease, and the importance of the interaction between cancer cells and inflammatory responses occurring in immune cells and basal cells of the tumor microenvironment in cancer progression have been confirmed, and numerous research and development results on the effectiveness of neurological disease and cancer treatment strategies through the resolution of inflammation have been reported, these strategies are considered new approaches. Particularly, clinical trials of FPR2-targeting peptides and small molecule compounds are underway for atopic dermatitis, heat failure, myocardial infarction, cardiovascular thrombosis, Crohn's disease, etc.

The present inventors developed a peptide with antibacterial activity in previous research and secured a patent (Patent Document 1), and developed a peptide with excellent antibacterial and immune function-regulating abilities and secured a patent (Patent Document 2).

### [Related Art Documents]

### [Patent Documents]

Patent Document 1 : Korean Patent No. 10-1855170
Patent Document 2 : Korean Patent No. 10-2170236

### [Disclosure]

### [Technical Problem]

The present invention is directed to providing a novel peptide having an immunoregulatory function and therapeutic effect on immune diseases by inducing the resolution of inflammation.

The present invention is also directed to providing various uses of the peptide, such as a pharmaceutical composition for preventing or treating an immune disease or cancer, and an anti-inflammatory cosmetic composition.

### [Technical Solution]

To achieve the above purposes, one aspect of the present invention provides a peptide consisting of an amino acid sequence represented by X1-X2-X3-X4-X5-X6-m,
wherein X1 is absent, or arginine (R), lysine (K), histidine (H), or proline (P); X2 is methionine (M) or tryptophan (W); X3 is a basic amino acid such as arginine (R) or lysine (K); X4 is tyrosine (Y); X5 is a basic amino acid such as arginine (R) or lysine (K), or an aromatic amino acid such as tryptophan (W) or tyrosine (Y); X6 is proline (P), tyrosine (Y), or valine (V); and m is D-methionine.

Another aspect of the present invention provides a peptide consisting of an amino acid sequence represented by X2-X3-X4-X5-X6-m,
wherein X2 is isoleucine (I), proline (P), or histidine (H); X3 is lysine (K); X4 is tyrosine (Y) or phenylalanine (F); X5 is lysine (K) or tryptophan (W); X6 is valine (V), proline (P), tyrosine (Y), or methionine (M); and m is D-methionine.

Still another aspect of the present invention provides a peptide consisting of an amino acid sequence represented by X1-X2-X3-X4-X5-X6-m,
wherein X1 is a basic amino acid such as arginine (R) or lysine (K); X2 is an aromatic amino acid such as tryptophan (W); X3 a basic amino acid such as arginine (R) or lysine (K); X4 is tyrosine (Y); X5 is arginine (R), lysine (K), leucine (L), asparagine (N), phenylalanine (F), or threonine (T); X6 is isoleucine (I), threonine (T), tyrosine (Y), or valine (V); and m is D-methionine.

Yet another aspect of the present invention provides a pharmaceutical composition for preventing or treating an immune disease, including the above-described peptide as an active ingredient.

Yet another aspect of the present invention provides a pharmaceutical composition for preventing or treating cancer, including the above-described peptide as an active ingredient.

Yet another aspect of the present invention provides an anti-inflammatory cosmetic composition, including the above-described peptide as an active ingredient.

### [Advantageous Effects]

Since a novel peptide according to the present invention has the function of regulating the activity of immune cells, and an immune disease or cancer treatment function induced by the resolution of inflammation, it can be used as a pharmaceutical composition for treating an immune disease or cancer, or an anti-inflammatory cosmetic composition including the above-described peptide.

### [Description of Drawings]

FIG. 1 shows the results of analyzing the ability of peptides to inhibit the production of inflammatory cytokines at the individual level.
FIG. 2 shows the results of analyzing the efficacy of peptides in improving severity scores for collagen-induced arthritis.
FIG. 3 shows the results of analyzing the corneal damage (A) and corneal opacity score (B) according to peptide administration in a BAC-induced dry eye model.
FIG. 4 shows the results of analyzing the body weight (A) and colon length (B) according to peptide administration in a DSS-induced colitis model.
FIG. 5 shows the results of analyzing the ability of a peptide to inhibit cytokine expression in an SK-N-MC neuronal cell line (A) and a rat DRG neuron (B).
FIG. 6 shows the results of analyzing the ability of a peptide to improve the density of dopamine neuronal cells at the nerve terminal (A) and the number of neuronal cells (B) in an MPTP-induced Parkinson's disease model.
FIG. 7 shows the results of analyzing the efficacy of peptides in inhibiting tumor growth.

### [Modes of the Invention]

Hereinafter, the present invention will be described in detail.

The present invention provides a novel peptide that binds to an FPR2 receptor to perform the function of controlling an inflammatory response.

In one aspect of the present invention, a peptide consisting of an amino acid sequence represented by X1-X2-X3-X4-X5-X6-m is provided, wherein

X1 is absent, or arginine (R), lysine (K), histidine (H), or proline (P); X2 is methionine (M) or tryptophan (W); X3 is a basic amino acid such as arginine (R) or lysine (K); X4 is tyrosine (Y); X5 is a basic amino acid such as arginine (R) or lysine (K), or an aromatic amino acid such as tryptophan (W) or tyrosine (Y); X6 is proline (P), tyrosine (Y), or valine (V); and m is D-methionine.

The term "peptide" used herein refers to a polymer consisting of one or more amino acids connected by an amide bond (or a peptide bond), and includes pharmaceutically acceptable salts of peptides as well as the peptides. Conventionally, the amino end (or N-terminus) of a peptide is shown at the left end of the amino acid sequence of the peptide, and the carboxyl end (or C-terminus) is shown at the right end of the amino acid sequence thereof. The amino acid sequence of a peptide may be written with one-letter symbols to indicate amino acids covalently bonded by peptide bonds. When the D-conformation is not specifically indicated, the L-conformation may be assumed, and the amino acid residues constituting the peptide may be natural or non-natural amino acid residues.

The peptide of the present invention may be obtained by various methods well known in the art. For example, after extracting a protein from the living body, it may be treated with a proteolytic enzyme to reduce a molecular weight, or may be biologically produced using genetic recombination and protein expression systems, or may be produced by in vitro synthesis through chemical synthesis such as peptide synthesis or cell-free protein synthesis.

In one embodiment of the present invention, X1 may be absent, or arginine (R), lysine (K), histidine (H), or proline (P); X2 may be methionine (M) or tryptophan (W); X3 may be a basic amino acid such as arginine (R) or lysine (K); X4 may be tyrosine (Y); X5 may be a basic amino acid such as arginine (R) or lysine (K); X6 may be proline (P), tyrosine (Y), or valine (V); and m may be D-methionine.

In one embodiment of the present invention, X1 may be absent, or arginine (R), lysine (K); X2 may be methionine (M) or tryptophan (W); X3 may be a basic amino acid such as arginine (R) or lysine (K); X4 may be tyrosine (Y); X5 may be an aromatic amino acid such as tryptophan (W); X6 may be proline (P), tyrosine (Y), or valine (V); and m may be D-methionine.

In one embodiment of the present invention, X1 may be absent, or arginine (R), lysine (K); X2 may be methionine (M) or tryptophan (W); X3 may be a basic amino acid such as arginine (R); X4 may be tyrosine (Y); X5 may be an aromatic amino acid such as tyrosine (Y); X6 may be proline (P), tyrosine (Y), or valine (V); and m may be D-methionine.

In one embodiment of the present invention, X1 may be absent, or arginine (R), lysine (K), histidine (H), or proline (P); X2 may be methionine (M) or tryptophan (W); X3 may be a basic amino acid such as arginine (R) or lysine (K); X4 may be tyrosine (Y); X5 may be a basic amino acid such as lysine (K); X6 may be proline (P), tyrosine (Y), or valine (V); and m may be D-methionine.

In one embodiment of the present invention, X1 may be absent or arginine (R); X2 may be methionine (M) or tryptophan (W); X3 may be a basic amino acid such as arginine (R) or lysine (K); X4 may be tyrosine (Y); X5 may be an aromatic amino acid such as tryptophan (W); X6 may be proline (P), tyrosine (Y), or valine (V); and m may be D-methionine.

In one embodiment of the present invention, X1 may be absent, or arginine (R); X2 may be methionine (M) or tryptophan (W); X3 may be a basic amino acid such as arginine (R); X4 may be tyrosine (Y); X5 may be an aromatic amino acid such as tyrosine (Y); X6 may be proline (P), tyrosine (Y), or valine (V); and m may be D-methionine.

In the present invention, to obtain better chemical stability, enhanced pharmacological properties (half-life, absorption, potency, efficacy, etc.), altered specificity (e.g., wide spectrum of biological activity), and reduced antigenicity, one or more amino acids of the peptide may be chemically modified. The "stability" is used to include not only in vivo stability, which protects the peptide of the present invention from the in vivo attack of proteolytic enzymes, but also storage stability (e.g., room temperature storage stability). For example, a protecting group may be added to the N-terminus or C-terminus of the peptide of the present invention. Preferably, the protecting group may be an acetyl group, a butanoyl group, a hexanoyl group, an octanoyl group, a fluorenylmethoxycarbonyl group, a palmitoyl group, myristoyl group, a stearyl group, a butoxycarbonyl group, an acryloxycarbonyl group, or polyethylene glycol (PEG), but may be any component that can modify the properties of a peptide, and particularly, improve the stability of a peptide without limitation.

In the present invention, in the N-terminal amino acid of a peptide, the terminal amino group (H₂N-) may be alkylated or acylated. For example, the N-terminal amino group of a peptide may be acylated to include an aliphatic acyl group (i.e., including an acetyl group, a myristoyl group, or a butanoyl group), carboxylic acid, benzoic acid, trifluoroacetic acid, or sulfonic acid. In another example, the N-terminal amino acid of a peptide may be alkylated with aliphatic alkyl, halide, or aliphatic alkynesulfonic acid ester.

In the present invention, the C-terminal carboxyl group (-COOH) of a peptide may be amidated or esterified. For example, the C-terminal carboxyl group may be chemically modified by forming an amide with an amine. In another example, the C-terminal carboxyl group may be chemically modified by forming an ester with alcohol.

In addition, in the present invention, a side chain in an amino acid of a peptide may be chemically modified. For example, the phenyl group in tyrosine may be substituted with an aliphatic alkyl group, an aliphatic carboxyl group, or an alkoxy group, but the present invention is not limited thereto. The epsilon amino group in lysine may be chemically modified by forming an amide with, for example, an aliphatic carboxyl group, a benzoic acid group, or an amino acid group. Moreover, the epsilon amino group in lysine may be chemically modified by the alkylation of one or two C1 to C4 aliphatic alkyl groups, but the present invention is not limited thereto.

In one embodiment of the present invention, any one selected from the group consisting of an acetyl group, a butanoyl group, a hexanoyl group, and an ocatonyl group may be added to the N-terminus of the peptide.

In one embodiment of the present invention, the C-terminus of the peptide may be amidated.

In one embodiment of the present invention, the peptide may consist of any one of the amino acid sequences of SEQ ID NOs: 1 to 127.

In one aspect of the present invention, a peptide consisting of an amino acid sequence represented by X2-X3-X4-X5-X6-m is provided, wherein

X2 may be isoleucine (I), proline (P), or histidine (H); X3 may be lysine (K); X4 is tyrosine (Y) or phenylalanine (F); X5 may be lysine (K) or tryptophan (W); X6 may be valine (V), proline (P), tyrosine (Y), or methionine (M); and m may be D-methionine.

In one embodiment of the present invention, X2 may be isoleucine (I), proline (P), or histidine (H); X3 may be lysine (K); X4 is tyrosine (Y) or phenylalanine (F); X5 may be lysine (K) or tryptophan (W); X6 may be valine (V) or proline (P); and m may be D-methionine.

In one embodiment of the present invention, X2 may be isoleucine (I) or histidine (H); X3 may be lysine (K); X4 is tyrosine (Y) or phenylalanine (F); X5 may be lysine (K) or tryptophan (W); X6 may be tyrosine (Y) or methionine (M); and m may be D-methionine.

In one embodiment of the present invention, X2 may be isoleucine (I) or proline (P); X3 may be lysine (K); X4 may be tyrosine (Y) or phenylalanine (F); X5 may be lysine (K) or tryptophan (W); X6 may be valine (V); and m may be D-methionine.

In one embodiment of the present invention, X2 may be isoleucine (I) or histidine (H); X3 may be lysine (K); X4 may be tyrosine (Y) or phenylalanine (F); X5 may be lysine (K) or tryptophan (W); X6 may be proline (P); and m may be D-methionine.

In one embodiment of the present invention, X2 may be isoleucine (I); X3 may be lysine (K); X4 may be phenylalanine (F); X5 may be lysine (K) or tryptophan (W); X6 may be tyrosine (Y) or methionine (M); and m may be D-methionine.

In one embodiment of the present invention, X2 may be isoleucine (I) or proline (P); X3 may be lysine (K); X4 may be tyrosine (Y) or phenylalanine (F); X5 may be lysine (K); X6 may be valine (V); and m may be D-methionine.

In one embodiment of the present invention, X2 may be isoleucine (I) or histidine (H); X3 may be lysine (K); X4 may be tyrosine (Y) or phenylalanine (F); X5 may be lysine (K) or tryptophan (W); X6 may be proline (P); and m may be D-methionine.

In one embodiment of the present invention, X2 may be isoleucine (I); X3 may be lysine (K); X4 may be phenylalanine (F); X5 may be lysine (K) or tryptophan (W); X6 may be tyrosine (Y) or methionine (M); m may be D-methionine.

In one embodiment of the present invention, any one selected from the group consisting of an acetyl group, a butanoyl group, a hexanoyl group, and an octanoyl group may be added to the N-terminus of the peptide.

In one embodiment of the present invention, the C-terminus of the peptide may be amidated.

In one embodiment of the present invention, the peptide may consist of any one of the amino acid sequences of SEQ ID NOs: 128 to 151.

In one aspect of the present invention, a peptide consisting of an amino acid sequence represented by X1-X2-X3-X4-X5-X6-m is provided,
wherein X1 is a basic amino acid such as arginine (R) or lysine (K); X2 is an aromatic amino acid such as tryptophan (W); X3 is a basic amino acid such as arginine (R) or lysine (K); X4 is tyrosine (Y); X5 is arginine (R), lysine (K), leucine (L), asparagine (N), phenylalanine (F), or threonine (T); X6 is isoleucine (I), threonine (T), tyrosine (Y), or valine (V); and m is D-methionine.

In one embodiment of the present invention, X1 may be a basic amino acid such as arginine (R) or lysine (K); X2 may be an aromatic amino acid such as tryptophan (W); X3 may be a basic amino acid such as arginine (R) or lysine (K); X4 may be tyrosine (Y); X5 may be arginine (R) or lysine (K); X6 may be isoleucine (I) or threonine (T); and m may be D-methionine.

In one embodiment of the present invention, X1 may be a basic amino acid such as arginine (R) or lysine (K); X2 may be an aromatic amino acid such as tryptophan (W); X3 may be a basic amino acid such as arginine (R) or lysine (K); X4 may be tyrosine (Y); X5 may be leucine (L), asparagine (N), phenylalanine (F), or threonine (T); X6 may be tyrosine (Y) or valine (V); and m may be D-methionine.

In one embodiment of the present invention, X1 may be a basic amino acid such as arginine (R); X2 may be an aromatic amino acid such as tryptophan (W); X3 may be a basic amino acid such as arginine (R); X4 may be tyrosine (Y); X5 may be arginine (R) or lysine (K); X6 may be isoleucine (I) or threonine (T); and m may be D-methionine.

In one embodiment of the present invention, X1 may be a basic amino acid such as arginine (R); X2 may be an aromatic amino acid such as tryptophan (W); X3 may be a basic amino acid such as arginine (R); X4 may be tyrosine (Y); X5 may be leucine (L), asparagine (N), phenylalanine (F), or threonine (T); X6 may be tyrosine (Y) or valine (V); and m may be D-methionine.

In one embodiment of the present invention, any one selected from the group consisting of an acetyl group, a butanoyl group, a hexanoyl group, and an octanoyl group may be added to the N-terminus of the peptide.

In one embodiment of the present invention, the C-terminus of the peptide may be amidated.

In one embodiment of the present invention, the peptide may consist of any one of the amino acid sequences of SEQ ID NOs: 152 to 160.

In the present specification, the peptide may directly or indirectly act as an immunomodulatory peptide that plays an immunomodulatory role such as regulating the inflammatory response of immune cells, regulating the migration of immune cells, regulating the inflammatory response of epidermal or endothelial cells. In the present invention, the immunomodulatory peptide may exhibit an immunomodulatory function by inducing the resolution of inflammation through the activation of formyl peptide receptor 2 (FPR2) expressed in phagocytic cells such as neutrophils and monocytes, antigen presenting cells such as dendritic cells, immune cells such as eosinophils, basophils, mast cells, and microglial cells, neuronal cells, epidermal cells, and endothelial cells.

As confirmed in the following experimental examples, in the case of the peptide according to the present invention, the FPR2 activation efficacy was shown to be high even at a low concentration, and selectivity was also confirmed to be high when compared with FPR1. In addition, as a result of measuring the change in the expression of cytokine genes in cells upon inflammation, the present inventors confirmed that inflammatory cytokines (TNFα, IL-6, and IL-1β) were lower than those of the control, and the efficacy of the peptide of the present invention in inhibiting inflammatory responses at the individual level was exhibited in an animal model.

Therefore, the present invention provides a pharmaceutical composition for preventing or treating an immune disease, including the above-described peptide as an active ingredient, a use of the peptide for preventing or treating an immune disease, and a method of preventing or treating an immune disease, which includes administering the peptide to a subject.

In one embodiment of the present invention, the immune disease may be atopic dermatitis, psoriasis, infantile eczema, sepsis, conjunctivitis, keratitis, dry eye syndrome, asthma, lung injury, lung inflammation, rheumatoid arthritis, ankylosing spondylitis, ulcerative colitis, Crohn's disease, peritonitis, systemic sclerosis, neuroinflammation, Parkinson's disease, or Alzheimer's disease, but the present invention is not limited thereto.

In the present invention, the peptide may act as a ligand that binds to FPR2 and may be used as a pharmaceutical composition for preventing or treating atopic dermatitis. For further details, refer to Baxendell et al. J Immunol May 1, 2018, 200 (1 Supplement) 105.4.

In the present invention, the peptide may act as a ligand that binds to FPR2 and may be used in a pharmaceutical composition for preventing or treating psoriasis. For further details, refer to Liu et al. Sci Rep. 2017 Aug 2;7(1):7100. doi: 10.1038/s41598-017-07485-1.

In the present invention, the peptide may act as a ligand that binds to FPR2 and may be used in a pharmaceutical composition for preventing or treating infantile eczema. For further details, refer to Wu et al. Br J Dermatol. 2013 Jan;168(1):172-8. doi: 10.1111/j.1365-2133.2012.11177.x.

In the present invention, the peptide may act as a ligand that binds to FPR2 and may be used in a pharmaceutical composition for preventing or treating sepsis. For further details, refer to Walker et al. SHOCK, Vol. 36, No. 4, pp. 410Y416, 2011.

In the present invention, the peptide may act as a ligand that binds to FPR2 and may be used in a pharmaceutical composition for preventing or treating conjunctivitis. For further details, refer to Hodges et al. Mucosal Immunol. 2017 Jan;10(1):46-57. doi: 10.1038/mi.2016.33. Epub 2016 Apr 13.

In the present invention, the peptide may act as a ligand that binds to FPR2 and may be used in a pharmaceutical composition for preventing or treating keratitis. For further details, refer to Zhu et al. Int Immunopharmacol. 2021 Jul;96:107785. doi: 10.1016/j.intimp.2021.107785. Epub 2021 May 24.

In the present invention, the peptide may act as a ligand that binds to FPR2 and may be used in a pharmaceutical composition for preventing or treating dry eye syndrome. For further details, refer to Gao et al. J Immunol. 2015 Oct 1;195(7):3086-99. doi: 10.4049/jimmunol.1500610. Epub 2015 Aug 31.

In the present invention, the peptide may act as a ligand that binds to FPR2 and may be used in a pharmaceutical composition for preventing or treating asthma. For further details, refer to Barnig et al. Eur Respir Rev. 2015 Mar;24(135):141-53. doi: 10.1183/09059180.00012514.

In the present invention, the peptide may act as a ligand that binds to FPR2 and may be used in a pharmaceutical composition for preventing or treating lung injury. For further details, refer to Ba et al. J Thorac Dis. 2019 Aug;11(8):3599-3608. doi: 10.21037/jtd.2019.08.86.

In the present invention, the peptide may act as a ligand that binds to FPR2 and may be used in a pharmaceutical composition for preventing or treating lung inflammation. For further details, refer to Sekheri et al. Proc Natl Acad Sci U S A. 2020 Apr 7;117(14):7971-7980. doi: 10.1073/pnas.1920193117. Epub 2020 Mar 23.

In the present invention, the peptide may act as a ligand that binds to FPR2 and may be used in a pharmaceutical composition for preventing or treating rheumatoid arthritis. For further details, refer to Kao et al. Br J Pharmacol. 2014 Sep;171(17):4087-96. doi: 10.1111/bph.12768.

In the present invention, the peptide may act as a ligand that binds to FPR2 and may be used in a pharmaceutical composition for preventing or treating ankylosing spondylitis. For further details, refer to Kao et al. Br J Pharmacol. 2014 Sep;171(17):4087-96. doi: 10.1111/bph.12768.

In the present invention, the peptide may act as a ligand that binds to FPR2 and may be used in a pharmaceutical composition for preventing or treating ulcerative colitis or Crohn's disease. For further details, refer to Kim et al. Exp Mol Med. 2013 Sep 13;45(9):e40. doi: 10.1038/emm.2013.77.

In the present invention, the peptide may act as a ligand that binds to FPR2 and may be used in a pharmaceutical composition for preventing or treating systemic sclerosis. For further details, refer to Park et al. Front Immunol. 2019 Sep 3;10:2095. doi: 10.3389/fimmu.2019.02095. eCollection 2019.

In the present invention, the peptide may act as a ligand that binds to FPR2 and may be used in a pharmaceutical composition for preventing or treating neuroinflammation. For further details, refer to Ponce et al. Front Aging Neurosci. 2022 Feb 17;14:780811. doi: 10.3389/fnagi.2022.780811. eCollection 2022.

In the present invention, the peptide may act as a ligand that binds to FPR2 and may be used in a pharmaceutical composition for preventing or treating Parkinson's disease. For further details, refer to Krashia et al. Nat Commun. 2019 Sep 2;10(1):3945. doi: 10.1038/s41467-019-11928-w.

In the present invention, the peptide may act as a ligand that binds to FPR2 and may be used in a pharmaceutical composition for preventing or treating Alzheimer's disease. For further details, refer to Pamplona et al. Transl Psychiatry. 2022 Oct 10;12(1):439. doi: 10.1038/s41398-022-02208-1.

In the present invention, the peptide may act as a pro-resolving factor binding to an FPR2 receptor.

In addition, the present inventors confirmed that tumor growth is inhibited when an animal model is treated with the peptide of the present invention. Therefore, it was confirmed that the peptide inducing the resolution of inflammation can inhibit tumor growth through immunomodulation.

Accordingly, the present invention provides a pharmaceutical composition for preventing or treating cancer, including the peptide as an active ingredient, a use of the peptide for preventing or treating cancer, and a method of preventing or treating cancer, which includes administering the peptide to a subject.

In one embodiment of the present invention, the cancer may be solid cancer or non-solid cancer, and may be gastric cancer, breast cancer, lung cancer, liver cancer, blood cancer, bone cancer, pancreatic cancer, skin cancer, head or neck cancer, cutaneous or intraocular melanoma, uterine sarcoma, ovarian cancer, rectal cancer, anal cancer, colon cancer, colorectal cancer, fallopian tube carcinoma, endometrial carcinoma, cervical cancer, small intestine cancer, endocrine cancer, thyroid cancer, parathyroid cancer, adrenal cancer, soft tissue tumors, urethral cancer, prostate cancer, bronchogenic cancer, bone marrow tumors, or lymphoma.

Preferably, the cancer may be breast cancer, colon cancer, colorectal cancer, lung cancer, ovarian cancer, pancreatic cancer, melanoma, or lymphoma, but the present invention is not limited thereto.

In the present invention, the peptide may act as a ligand that binds to FPR2 and may be used in a pharmaceutical composition for preventing or treating cancer. For further details, refer to Kim et al. PLoS One. 2012;7(1):e30522. doi: 10.1371/journal.pone.0030522. Epub 2012 Jan 25; Wang et al. Antioxidants (Basel). 2021 Sep 14;10(9):1459. doi: 10.3390/antiox10091459.; Liu et al. Cancer Res. 2013 Jan 15;73(2):550-60. doi: 10.1158/0008-5472.CAN-12-2290. Epub 2012 Nov 8; Gilligan et al. Proc Natl Acad Sci U S A. 2019 Mar 26;116(13):6292-6297. doi: 10.1073/pnas.1804000116. Epub 2019 Mar 12; Du et al. Ann Transl Med. 2021 May;9(9):802. doi: 10.21037/atm-21-1873. Erratum in: Ann Transl Med. 2022 Apr;10(7):428; Sulciner et al. J Exp Med. 2018 Jan 2;215(1):115-140. doi: 10.1084/jem.20170681. Epub 2017 Nov 30; Zhang et al. Front Immunol. 2017 Feb 2;8:71. doi: 10.3389/fimmu.2017.00071; Liotti et al. Cancers (Basel). 2022 Jul 8;14(14):3333. doi: 10.3390/cancers14143333.

The term "prevention" used herein refers to all actions involved in inhibiting or delaying an immune disease or cancer by administering the pharmaceutical composition according to the present invention, and the term "treatment" used herein refers to all actions involved in alleviating or beneficially changing an immune disease or cancer by administering the pharmaceutical composition according to the present invention. The "subject" used herein refers to an individual in need of prevention or treatment of a disease, and more specifically, can be any mammal, including, for example, not only a human or other primates, but also livestock including cattle, pigs, sheep, horses, dogs, and cats without limitation, but is preferably a human.

An administration route of the pharmaceutical composition including the peptide as an active ingredient may be any of the general routes that can deliver a drug to target tissue, and the pharmaceutical composition may be administered to a subject orally or parenterally. For example, the pharmaceutical composition may be provided by intraperitoneal, intravenous, intraarterial, intramuscular, subcutaneous, intradermal, oral, topical (including dermal application, instillation, and inhalation), transdermal, intrathecal and epidural, intranasal, intraocular, intrapulmonary, rectal, or intravaginal administration, but the present invention is not limited thereto. In addition, the pharmaceutical composition including the peptide as an active ingredient may be administered in any form of convenient pharmaceutical product, for example, tablets, powder, granules, capsules, an oral liquid, a solution, a dispersion, a suspension, a syrup, a spray, suppositories, a gel, an emulsion, or patches. However, since the peptide is digested during oral administration, an oral composition is preferably formulated to coat an active agent or to protect it from decomposition in the stomach.

The pharmaceutical composition including the peptide of the present invention may further include a pharmaceutically or physiologically acceptable carrier, excipient and diluent. The term "pharmaceutically acceptable carrier, excipient and diluent" used herein refer to a carrier, an excipient, and a diluent, which do not irritate a living organism and do not inhibit the biological activity and properties of the administered compound. Examples of appropriate carriers, excipients and diluents, which can be included in the composition, may include saline, sterile water, Ringer's solution, buffered saline, albumin injection solution, glycerol, ethanol, lactose, dextrose, sucrose, sorbitol, mannitol, xylitol, erythritol, maltitol, starch, gum acacia, alginate, gelatin, calcium phosphate, calcium silicate, cellulose, methyl cellulose, amorphous cellulose, hypromellose, polyvinylpyrrolidone, water, methylhydroxybenzoate, propylhydroxybenzoate, talc, magnesium stearate, and mineral oil. When the composition is pharmaceutically formulated, it may further include a conventional dispersant, filler, extender, binder, disintegrant, surfactant, anti-coagulant, lubricant, wetting agent, fragrance, emulsifier, preservative or freeze-drying agent.

In the present invention, the pharmaceutical composition may be administered in the form of an injection. The injection may be formulated as intravenous or subcutaneous injections. In the case of a parenteral injection, ingredients contained in a general injection composition may be included. For example, the injection composition includes a liquid carrier such as sterile water, water for injection, and physiological saline. In addition, the injection composition may further include an amino acid, a sugar, a lipid, a vitamin, an electrolyte, a pH adjuster, a stabilizer, an osmotic pressure adjuster, or a solubilizer.

In the present invention, when the pharmaceutical composition is topically administered, it may be formulated in the form of, for example, an ointment, a gel, a cream, or a lotion. The type of topical administration may be, but is not limited to, for example, the application to the skin, instillation into the eyes, transdermal penetration using microneedles, or intradermal injection. For example, the application of the composition or the attachment of a patch containing the composition is preferable. The composition may include, for example, a base material, an excipient, a lubricant, and a preservative. For example, when the pharmaceutical composition is administered in the form of eye drops, it may further include a buffer, a viscosity agent, an isotonic agent, a pH adjuster, or a solvent. In addition, when the pharmaceutical composition is administered in the form of an ointment for dermal application, it may further include a gelating agent, a stabilizer, an emulsifier, or a suspending agent.

In addition, the pharmaceutical composition of the present invention may be applied differently depending on the purpose of administration and disease. The amount of the active ingredient actually administered may be suitably selected by those of ordinary skill in the art, considering various related factors, such as a disease to be treated, the severity of the disease, whether it is co-administered with other drugs, the activity of a drug, the sensitivity to the drug, a patient's age, sex, weight, food, administration time, administration route, and the administration ratio of the composition. The composition may be administered once a day or in divided doses 1 to 3 times, although the dosage and administration route may be adjusted depending on the type and severity of the disease.

The pharmaceutical composition including the peptide of the present invention as an active ingredient may be administered at a dose of 0.001 mg/kg to 1 g/kg, and more preferably, 0.1 mg/kg to 100 mg/kg. However, the dose may be suitably adjusted according to a patient's age, sex, and condition.

In addition, the present invention provides an anti-inflammatory cosmetic composition including the peptide as an active ingredient, a use of the peptide for inhibiting inflammation, and a method of inhibiting inflammation, which includes applying the peptide on a subject.

The anti-inflammatory cosmetic composition including the peptide of the present invention may be prepared in a general emulsion formulation or solubilizing formulation. For example, the anti-inflammatory cosmetic composition may be formulated in the form of a solution, an ointment for external use, a toner such as a softening lotion or nourishing lotion, an emulsion such as a facial lotion or body lotion, a cream such as a nourishing cream, a moisturizing cream, or an eye cream, an essence, a cosmetic ointment, a spray, a gel, a pack, a sunscreen, a makeup base, a liquid-, solid- or spray-type foundation, a powder, a makeup remover such as a cleansing cream, a cleansing lotion, or a cleansing oil, a cleansing foam, a cleanser such as a soap or a body wash, an oil, or a patch, but the present invention is not limited thereto.

In the present invention, the cosmetic composition may further include one or more supplements and carriers, which are conventionally used in the field of cosmetology, such as a pearling agent, a thickener, a viscosity modifier, a pH adjuster, a fatty substance, an organic solvent, a solubilizer, a concentrating agent, a gelling agent, a softener, an anti-oxidant, a suspending agent, a stabilizer, a foaming agent, a fragrance a surfactant, water, an ionic or nonionic emulsifier, a filler, a chelating agent, a preservative, a vitamin, a blocking agent, a humectant, an essential oil, a dye, a pigment, a hydrophilic activator, a lipophilic activator, a lipid vesicle, or any other ingredients commonly used in cosmetics.

Hereinafter, the advantages and features of the present invention and the methods of accomplishing the same will become apparent with reference to the following examples to be described in detail and the accompanying drawings. However, the present invention is not limited to the exemplary embodiments disclosed below, and may be embodied in many different forms. These exemplary embodiments are merely provided to complete the disclosure of the present invention and fully convey the scope of the present invention to those of ordinary skill in the art, and the present invention should be defined by only the accompanying claims.

### Example 1: Preparation of immunomodulatory peptide

In the present invention, to synthesize a novel peptide having an excellent immunomodulatory function compared to the conventional one, a conventional amino acid synthesis method (Umbarger, H E, Ann Rev Biochem., 47: 533-606, 1978) was used, and the synthesized peptides were referred to as immunomodulatory candidate peptides. Moreover, the present inventors chemically modified the N-terminus and/or the C-terminus of the synthesized peptides. The synthesized peptide sequences are shown in Table 1.

**[Table1]**

| SEQ ID NO | Sequence | | | | | | | | |
|---|---|---|---|---|---|---|---|---|---|
| | N-terminus | X1 | N-terminus | X3 | N-terminus | X5 | N-terminus | m | N-terminus |
| 1 | | R | W | R | Y | K | V | m | NH₂ |
| 2 | Oct | R | W | R | Y | K | V | m | NH₂ |
| 3 | | | W | R | Y | K | V | m | NH₂ |
| 4 | Ac | K | W | K | Y | K | V | m | NH₂ |
| 5 | | R | M | R | Y | K | V | m | NH₂ |
| 6 | Ac | R | M | R | Y | K | V | m | NH₂ |
| 7 | But | R | M | R | Y | K | V | m | NH₂ |
| 8 | Oct | R | M | R | Y | K | V | m | NH₂ |
| 9 | | K | M | K | Y | K | V | m | NH₂ |
| 10 | Ac | K | M | K | Y | K | V | m | NH₂ |
| 11 | But | K | M | K | Y | K | V | m | NH₂ |
| 12 | Oct | K | M | K | Y | K | V | m | NH₂ |
| 13 | | | M | R | Y | K | V | m | NH₂ |
| 14 | Ac | | M | R | Y | K | V | m | NH₂ |
| 15 | But | | M | R | Y | K | V | m | NH₂ |
| 16 | Oct | | M | R | Y | K | V | m | NH₂ |
| 17 | | | M | K | Y | K | V | m | NH₂ |
| 18 | Ac | | M | K | Y | K | V | m | NH₂ |
| 19 | But | | M | K | Y | K | V | m | NH₂ |
| 20 | Oct | | M | K | Y | K | V | m | NH₂ |
| 21 | | H | M | K | Y | K | V | m | NH₂ |
| 22 | Ac | H | M | K | Y | K | V | m | NH₂ |
| 23 | But | H | M | K | Y | K | V | m | NH₂ |
| 24 | Oct | H | M | K | Y | K | V | m | NH₂ |
| 25 | | P | M | K | Y | K | V | m | NH₂ |
| 26 | Ac | P | M | K | Y | K | V | m | NH₂ |
| 27 | But | P | M | K | Y | K | V | m | NH₂ |
| 28 | Oct | P | M | K | Y | K | V | m | NH₂ |
| 29 | | R | W | R | Y | R | V | m | NH₂ |
| 30 | | R | M | R | Y | R | V | m | NH₂ |
| 31 | | H | M | R | Y | R | V | m | NH₂ |
| 32 | | R | W | R | Y | K | Y | m | NH₂ |
| 33 | Ac | R | W | R | Y | K | Y | m | NH₂ |
| 34 | Oct | R | W | R | Y | K | Y | m | NH₂ |
| 35 | | | W | R | Y | K | Y | m | NH₂ |
| 36 | Ac | K | W | K | Y | K | Y | m | NH₂ |
| 37 | But | H | W | K | Y | K | Y | m | NH₂ |
| 38 | | R | M | R | Y | K | Y | m | NH₂ |
| 39 | Ac | R | M | R | Y | K | Y | m | NH₂ |
| 40 | Oct | R | M | R | Y | K | Y | m | NH₂ |
| 41 | | | M | R | Y | K | Y | m | NH₂ |
| 42 | Ac | | M | R | Y | K | Y | m | NH₂ |
| 43 | But | | M | R | Y | K | Y | m | NH₂ |
| 44 | | H | M | R | Y | K | Y | m | NH₂ |
| 45 | But | H | M | R | Y | K | Y | m | NH₂ |
| 46 | | P | M | R | Y | K | Y | m | NH₂ |
| 47 | But | P | M | R | Y | K | Y | m | NH₂ |
| 48 | | R | W | R | Y | R | Y | m | NH₂ |
| 49 | Ac | | M | R | Y | R | Y | m | NH₂ |
| 50 | | R | M | R | Y | K | P | m | NH₂ |
| 51 | Ac | R | M | R | Y | K | P | m | NH₂ |
| 52 | But | R | M | R | Y | K | P | m | NH₂ |
| 53 | Oct | R | M | R | Y | K | P | m | NH₂ |
| 54 | | | M | R | Y | K | P | m | NH₂ |
| 55 | Ac | | M | R | Y | K | P | m | NH₂ |
| 56 | But | | M | R | Y | K | P | m | NH₂ |
| 57 | Oct | | M | R | Y | K | P | m | NH₂ |
| 58 | | H | M | R | Y | K | P | m | NH₂ |
| 59 | Ac | H | M | R | Y | K | P | m | NH₂ |
| 60 | But | H | M | R | Y | K | P | m | NH₂ |
| 61 | Oct | H | M | R | Y | K | P | m | NH₂ |
| 62 | | P | M | R | Y | K | P | m | NH₂ |
| 63 | Ac | P | M | R | Y | K | P | m | NH₂ |
| 64 | But | P | M | R | Y | K | P | m | NH₂ |
| 65 | Oct | P | M | R | Y | K | P | m | NH₂ |
| 66 | | K | M | K | Y | K | P | m | NH₂ |
| 67 | Ac | K | M | K | Y | K | P | m | NH₂ |
| 68 | But | K | M | K | Y | K | P | m | NH₂ |
| 69 | Oct | K | M | K | Y | K | P | m | NH₂ |
| 70 | | | M | K | Y | K | P | m | NH₂ |
| 71 | Ac | | M | K | Y | K | P | m | NH₂ |
| 72 | But | | M | K | Y | K | P | m | NH₂ |
| 73 | Oct | | M | K | Y | K | P | m | NH₂ |
| 74 | | R | W | R | Y | K | P | m | NH₂ |
| 75 | But | R | W | R | Y | K | P | m | NH₂ |
| 76 | Ac | | W | R | Y | K | P | m | NH₂ |
| 77 | | R | W | R | Y | W | Y | m | NH₂ |
| 78 | Ac | R | W | R | Y | W | Y | m | NH₂ |
| 79 | | | W | R | Y | W | Y | m | NH₂ |
| 80 | Ac | K | W | K | Y | W | Y | m | NH₂ |
| 81 | | R | M | R | Y | W | Y | m | NH₂ |
| 82 | Ac | R | M | R | Y | W | Y | m | NH₂ |
| 83 | But | R | M | R | Y | W | Y | m | NH₂ |
| 84 | Oct | R | M | R | Y | W | Y | m | NH₂ |
| 85 | | | M | R | Y | W | Y | m | NH₂ |
| 86 | Ac | | M | R | Y | W | Y | m | NH₂ |
| 87 | But | | M | R | Y | W | Y | m | NH₂ |
| 88 | Oct | | M | R | Y | W | Y | m | NH₂ |
| 89 | | | M | K | Y | W | Y | m | NH₂ |
| 90 | Ac | | M | K | Y | W | Y | m | NH₂ |
| 91 | But | | M | K | Y | W | Y | m | NH₂ |
| 92 | Ac | H | M | R | Y | W | Y | m | NH₂ |
| 93 | But | H | M | R | Y | W | Y | m | NH₂ |
| 94 | | R | W | R | Y | Y | V | m | NH₂ |
| 95 | Ac | | W | R | Y | Y | V | m | NH₂ |
| 96 | | K | W | K | Y | Y | V | m | NH₂ |
| 97 | | R | M | R | Y | Y | V | m | NH₂ |
| 98 | Ac | R | M | R | Y | Y | V | m | NH₂ |
| 99 | Oct | R | M | R | Y | Y | V | m | NH₂ |
| 100 | | | M | R | Y | Y | V | m | NH₂ |
| 101 | | R | W | R | Y | W | V | m | NH₂ |
| 102 | Ac | | W | R | Y | W | V | m | NH₂ |
| 103 | Ac | K | W | K | Y | W | V | m | NH₂ |
| 104 | Ac | R | M | R | Y | W | V | m | NH₂ |
| 105 | Ac | | M | R | Y | W | V | m | NH₂ |
| 106 | | R | M | R | Y | W | P | m | NH₂ |
| 107 | Ac | R | M | R | Y | W | P | m | NH₂ |
| 108 | But | R | M | R | Y | W | P | m | NH₂ |
| 109 | Oct | R | M | R | Y | W | P | m | NH₂ |
| 110 | | | M | R | Y | W | P | m | NH₂ |
| 111 | Ac | | M | R | Y | W | P | m | NH₂ |
| 112 | But | | M | R | Y | W | P | m | NH₂ |
| 113 | | | M | K | Y | W | P | m | NH₂ |
| 114 | Ac | | M | K | Y | W | P | m | NH₂ |
| 115 | But | | M | K | Y | W | P | m | NH₂ |
| 116 | Oct | | M | K | Y | W | P | m | NH₂ |
| 117 | Ac | R | W | R | Y | W | P | m | NH₂ |
| 118 | | | W | R | Y | W | P | m | NH₂ |
| 119 | Ac | R | M | R | Y | Y | P | m | NH₂ |
| 120 | | | M | R | Y | Y | P | m | NH₂ |
| 121 | Ac | R | W | R | Y | Y | P | m | NH₂ |
| 122 | Ac | | W | R | Y | Y | P | m | NH₂ |
| 123 | | R | W | R | Y | Y | Y | m | NH₂ |
| 124 | Ac | | W | R | Y | Y | Y | m | NH₂ |
| 125 | Ac | R | M | R | Y | Y | Y | m | NH₂ |
| 126 | Ac | | M | R | Y | Y | Y | m | NH₂ |
| 127 | But | H | M | K | Y | Y | Y | m | NH₂ |
| 128 | | | I | K | F | W | Y | m | NH₂ |
| 129 | | | I | K | F | W | M | m | NH₂ |
| 130 | | | I | K | K | W | M | m | NH₂ |
| 131 | | | I | K | F | K | Y | m | NH₂ |
| 132 | | | I | K | F | K | V | m | NH₂ |
| 133 | | | I | K | F | K | P | m | NH₂ |
| 134 | | | I | K | Y | K | V | m | NH₂ |
| 135 | Ac | | I | K | Y | K | V | m | NH₂ |
| 136 | But | | I | K | Y | K | V | m | NH₂ |
| 137 | Oct | | I | K | Y | K | V | m | NH₂ |
| 138 | | | P | K | Y | K | V | m | NH₂ |
| 139 | Ac | | P | K | Y | K | V | m | NH₂ |
| 140 | But | | P | K | Y | K | V | m | NH₂ |
| 141 | Oct | | P | K | Y | K | V | m | NH₂ |
| 142 | | | I | K | Y | K | P | m | NH₂ |
| 143 | Ac | | I | K | Y | K | P | m | NH₂ |
| 144 | But | | I | K | Y | K | P | m | NH₂ |
| 145 | Oct | | I | K | Y | K | P | m | NH₂ |
| 146 | | | H | K | Y | K | P | m | NH₂ |
| 147 | Ac | | H | K | Y | K | P | m | NH₂ |
| 148 | But | | H | K | Y | K | P | m | NH₂ |
| 149 | Oct | | H | K | Y | K | P | m | NH₂ |
| 150 | Ac | | H | K | Y | W | P | m | NH₂ |
| 151 | But | | H | K | Y | W | P | m | NH₂ |
| 152 | | R | W | R | Y | R | I | m | NH₂ |
| 153 | | R | W | R | Y | R | T | m | NH₂ |
| 154 | | R | W | R | Y | L | Y | m | NH₂ |
| 155 | | R | W | R | Y | N | Y | m | NH₂ |
| 156 | | R | W | R | Y | F | Y | m | NH₂ |
| 157 | | R | W | R | Y | T | Y | m | NH₂ |
| 158 | | R | W | R | Y | L | V | m | NH₂ |
| 159 | | R | W | R | Y | N | V | m | NH₂ |
| 160 | | R | W | R | Y | F | V | m | NH₂ |

### Experimental Example 1: Measurement of change in calcium activity by peptide

The present inventors observed changes in calcium ion permeability to confirm whether the immunomodulatory peptide candidates prepared according to one example of the present invention activate the immune function of a living organism. Specifically, the concentration of calcium ions in cells was measured to confirm whether the peptides activate FPR2.

### 1) Preparation of cells and peptides

The present inventors used RBL cells that do not express FPR2 (RBL-WT) and RBL cells in which FPR2 was overexpressed (RBL-FPR2), and as a method for sensitively measuring the release of intracellular calcium ions, Fura-2/AM, which is a staining substance having a strong binding strength to calcium, was used.

A peptide dissolved in water or 50% DMSO at a concentration of 0.5mM was prepared by diluting it to an appropriate concentration in HBSS (Sigma, #55037C) + 20 mM HEPES buffer.

### 2) Culture conditions

In this experiment, the cells were cultured in DMEM (Lonza, #BE12-604F) containing 10% fetal bovine serum (FBS) and 1% penicillin-streptomycin under conditions of 37 °C and 5% CO₂.

### 3) Experimental method

The day before measuring calcium activity, the cells were prepared at 3 x 10⁵ cells per well in a 96-well flat clear bottom black plate (Costar, #3603). The prepared cells were treated with a Fura-2 QBT calcium kit (component A; Molecular Devices, #8197) at the same volume per well. After incubation at 37 °C for 1 hour, the cells were incubated in a dark room at room temperature for 30 minutes. After the Fura-2-treated cells, the prepared peptide, and a dedicated tip were placed at each section of FlexStation 3 Multi-Mode Microplate Reader (Molecular Devices, #Flex3), changes in calcium activity caused by the peptides were measured using SoftMax Pro 7 Software (Molecular Devices, #FLEX3-INSTALL-OS).

### 4) Experimental results

As a result, most peptides showed an FPR2 activation effect, suggesting that the peptide according to the present invention can regulate the immune function of an individual through binding to and activating FPR2. The results of analyzing the FPR2 activation effect of the peptide were graded according to numerical values and summarized in Table 2 below. The criteria for each grade are described in Table 3.

**[Table2]**

| Analysis of FPR2 and FPR1 activation effect of peptides | | |
|---|---|---|
| SEQ ID NO. | EC50for[Ca²⁺] | |
| | FPR2(nM) | FPR1(µM) |
| 1 | A | E |
| 2 | B | D |
| 3 | A | E |
| 4 | A | E |
| 5 | A | E |
| 6 | A | E |
| 7 | A | E |
| 8 | B | E |
| 9 | B | E |
| 10 | B | E |
| 11 | A | E |
| 12 | B | E |
| 13 | A | E |
| 14 | A | D |
| 15 | A | E |
| 16 | B | E |
| 17 | A | E |
| 18 | A | E |
| 19 | A | E |
| 20 | B | E |
| 21 | A | E |
| 22 | A | E |
| 23 | A | E |
| 24 | A | E |
| 25 | A | E |
| 26 | A | E |
| 27 | A | E |
| 28 | A | E |
| 29 | A | E |
| 30 | A | E |
| 31 | A | D |
| 32 | B | E |
| 33 | A | E |
| 34 | B | E |
| 35 | A | E |
| 36 | A | E |
| 37 | A | E |
| 38 | A | E |
| 39 | B | E |
| 40 | B | E |
| 41 | A | E |
| 42 | B | E |
| 43 | B | E |
| 44 | B | E |
| 45 | A | E |
| 46 | A | E |
| 47 | A | E |
| 48 | B | E |
| 49 | B | E |
| 50 | A | E |
| 51 | A | E |
| 52 | A | E |
| 53 | A | E |
| 54 | A | E |
| 55 | A | E |
| 56 | A | E |
| 57 | A | E |
| 58 | A | E |
| 59 | A | E |
| 60 | A | E |
| 61 | A | E |
| 62 | A | E |
| 63 | A | E |
| 64 | A | E |
| 65 | A | E |
| 66 | B | E |
| 67 | A | E |
| 68 | A | E |
| 69 | B | E |
| 70 | A | E |
| 71 | B | E |
| 72 | B | E |
| 73 | B | E |
| 74 | A | E |
| 75 | A | D |
| 76 | A | D |
| 77 | B | E |
| 78 | A | E |
| 79 | B | E |
| 80 | A | E |
| 81 | A | E |
| 82 | A | E |
| 83 | A | E |
| 84 | B | E |
| 85 | A | E |
| 86 | A | E |
| 87 | A | E |
| 88 | B | E |
| 89 | A | E |
| 90 | B | E |
| 91 | B | E |
| 92 | A | E |
| 93 | A | D |
| 94 | A | D |
| 95 | B | C |
| 96 | A | D |
| 97 | A | D |
| 98 | A | D |
| 99 | B | D |
| 100 | A | D |
| 101 | A | D |
| 102 | A | C |
| 103 | B | D |
| 104 | A | D |
| 105 | A | C |
| 106 | A | C |
| 107 | A | C |
| 108 | A | C |
| 109 | A | D |
| 110 | A | C |
| 111 | A | D |
| 112 | A | D |
| 113 | A | D |
| 114 | A | D |
| 115 | A | D |
| 116 | B | D |
| 117 | A | C |
| 118 | A | C |
| 119 | A | C |
| 120 | A | C |
| 121 | A | C |
| 122 | A | C |
| 123 | A | E |
| 124 | B | D |
| 125 | A | E |
| 126 | B | E |
| 127 | A | E |
| 128 | B | E |
| 129 | B | E |
| 130 | B | E |
| 131 | B | E |
| 132 | B | E |
| 133 | A | E |
| 134 | A | E |
| 135 | A | E |
| 136 | A | E |
| 137 | A | D |
| 138 | A | E |
| 139 | A | E |
| 140 | A | E |
| 141 | A | E |
| 142 | A | E |
| 143 | A | E |
| 144 | A | E |
| 145 | B | E |
| 146 | A | E |
| 147 | A | E |
| 148 | A | E |
| 149 | A | E |
| 150 | A | D |
| 151 | A | D |
| 152 | B | E |
| 153 | B | E |
| 154 | A | E |
| 155 | B | E |
| 156 | A | E |
| 157 | A | E |
| 158 | B | D |
| 159 | B | D |
| 160 | B | D |

**[Table3]**

| Grade | |
|---|---|
| A | <10nM |
| B | 11~100nM |
| C | 101~1,000nM |
| D | 1,001~10,000nM |
| E | 10,001< |

As shown in Table 2, it was confirmed that the immunomodulatory peptide according to the present invention has an EC₅₀ value for FPR2 that is generally 100 nM or less, but an EC₅₀ value for FPR1 of 10,000 nM or more. Through this, it was confirmed that the immunomodulatory peptide specifically binds to FPR2.

### Experimental Example 2: Measurement of cytokine inhibition ability by peptide

The present inventors observed the cytokine inhibition ability of the immunomodulatory peptide candidates prepared according to one example of the present invention. Specifically, to confirm whether the peptides reduce inflammatory responses in cells, changes in cytokine gene expression in cells upon inflammation was measured.

### 1) Preparation of cells and peptides

The present inventors used a RAW264.7 cell line, that is, mouse macrophages, and used lipopolysaccharide (LPS; Sigma-Aldrich, #L2630), which is the main component of a gram-negative bacterial cell wall, as a material causing an inflammatory response. A peptide that had been dissolved in water at 100 µM was prepared.

### 2) Culture conditions

In the present experiment, the cells were cultured in a DMEM (Lonza, #BE12-604F) containing 10% FBS and 1% penicillin-streptomycin under conditions of 37 °C and 5% CO₂.

### 3) Experimental method

The cells were plated in a 24-well plate (Costar, #3603) at a density of 2.5 × 10⁵ cells/well. After 24 hours, the prepared cells were treated with the peptide at 100 nM. After incubation at 37 °C for 30 minutes, to induce inflammation, 200 ng/mL of LPS was treated. After incubation at 37 °C for 6 hours, the cells in a medium-free state were treated with TRIzol^{™} (Invitrogen, #15596018) to lyse the cells. Through the given experimental method, RNA was obtained, cDNA was produced using a reverse transcriptase (Promega, #M1705). Afterward, a cytokine expression level was measured using SYBR Green (Enzynomics, RT530) and mouse primers of each inflammatory cytokine gene (TNFα, IL-6, and IL-1β).

### 4) Experimental results

As a result, it was confirmed that, in most peptides, the inflammatory cytokines (TNFα, IL-6, and IL-1β) were reduced compared to the control. This shows that the peptide according to the present invention can reduce the inflammatory immune response in an individual. The results of analyzing changes in the expression of cytokine genes of the peptides were graded according to numerical values and summarized in Table 4 below. The criteria for each grade are described in Table 5.

**[Table 4]**

| Analysis of ability of peptide to inhibit cytokine expression | | | |
|---|---|---|---|
| | **Cytokine inhibition ability** | | |
| **Peptide** | **TNFα** | **IL-6** | **IL-1β** |
| 6 | A | C | A |
| 17 | B | D | B |
| 18 | A | D | A |
| 19 | B | B | A |
| 20 | B | B | B |
| 38 | B | C | A |
| 39 | B | D | B |
| 40 | B | C | A |
| 55 | B | B | A |
| 69 | B | C | A |
| 70 | C | D | A |
| 71 | C | C | A |
| 72 | B | D | B |
| 73 | B | D | B |
| 81 | A | C | A |
| 82 | A | D | B |
| 84 | A | E | B |
| 113 | D | B | D |
| 114 | A | E | B |
| 115 | C | B | B |
| 116 | B | B | B |

**[Table 5]**

| **Reduction rate(%)** | **Grade** |
|---|---|
| 81%-100% | A |
| 61%-80% | B |
| 41%-60% | C |
| 21%-40% | D |
| 0%-20% | E |
| <0% | F |

In the following experimental examples, a peptide having an excellent effect was selected from the candidates of the immunomodulatory peptides to confirm the efficacy of the peptide in an animal model.

### Experimental Example 3: Confirmation of peptide efficacy on inhibition of inflammatory response at individual level

The present inventors observed the efficacy on the inhibition of inflammatory response at the individual level using the immunomodulatory peptide candidates prepared according to one example of the present invention. Specifically, to confirm whether the peptides control inflammatory responses at the individual level, cytokine changes were observed in an LPS (Sigma-Aldrich, #L2630)-induced acute peritonitis model.

### 1) Method of inducing acute peritonitis model and administering peptide

The present inventors used approximately 6-week-old C57BL/6 mice, and LPS mixed with 5% mannitol (D-Mannitol; Sigma-Aldrich, #M8429) as a substance for inducing a peritoneal inflammatory disease. The inflammatory substance was injected intraperitoneally into the mice to induce a peritoneal inflammatory disease, and the peptides were intraperitoneally injected once immediately after the injection of the inflammatory substance.

### 2) Disease evaluation method

Blood and abdominal lavage fluid were collected 6 or 24 hours after the end of peptide administration, and an inflammatory factor such as a cytokine was analyzed using an enzyme-linked immunosorbent assay (ELISA) technique.

### 3) Experimental results

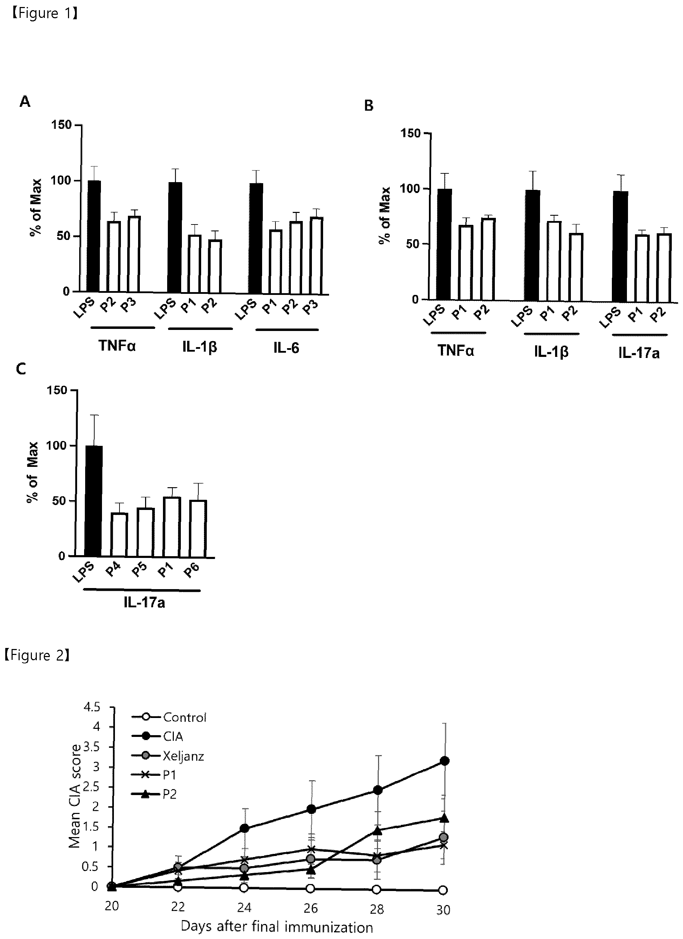

As a result, it was confirmed that, in most subjects, inflammatory cytokines (TNFα, IL-6, IL-1β, and IL-17a) were reduced compared to the control when the peptide was administered. This suggests that the peptide according to the present invention can suppress an inflammatory immune response at the individual level. The results of analyzing cytokine changes upon the administration of the peptide are shown in FIG. 1.

### Experimental Example 4: Confirmation of peptide efficacy on rheumatoid arthritis

The present inventors observed the efficacy on rheumatoid arthritis using the immunomodulatory peptide candidates prepared according to one example of the present invention. Specifically, to confirm whether the peptides reduce intracellular inflammatory responses, changes in clinical scores and arthritis symptoms such as sole edema were measured in a collagen-induced arthritis (CIA) model.

### 1) Method of inducing collagen-induced arthritis model and administering peptide

The present inventors used approximately 8 to 12-week-old DBA1/J mice, and Complete Freund's Adjuvant (CFA) and bovine type II collagen, which are inflammatory substances, were mixed and used as an arthritis-inducing substance. The inflammatory substance was injected into the mouse tail (1^{st} injection), and after about 2 to 3 weeks, the inflammatory substance was injected (2^{nd} boosting) to induce arthritis. Within several days, the peptide was injected intraperitoneally once every two days for a total of 10 times.

### 2) Method of evaluating improvement of rheumatoid arthritis

During the experimental period, body weights and visual changes were observed to confirm the overall health conditions of the animals. The severity scores for arthritis, and thickness changes due to sole and ankle edema were tracked and evaluated during the experimental period. After the final administration of the substance was completed, blood was collected and inflammatory factors such as cytokines were analyzed, tissue from the ankle area was removed to form a paraffin block, and tissue analysis was performed using hematoxylin and eosin (H&E) staining or immunohistochemistry.

### 3) Experimental results

As a result, it was confirmed that the severity score of collagen-induced arthritis (CIA) was improved when the peptide was administered. This suggests that the peptide according to the present invention can improve arthritis, which is an inflammatory disease, at the individual level. The results of analyzing the change in the severity score of the peptide are shown in FIG. 2, and the criteria for assigning the severity score are described in Table 6.

**[Table 6]**

| **Paw score** | **Clinical observations** |
|---|---|
| 0 | Normal paw. No noticeable difference in appearance, compared to healthy mice. |
| 1 | One or two toes are inflamed or swollen. No noticeable swelling in the paws or ankles. |
| 2 | Three or more toes are inflamed and swollen. However, no swelling in a paw or mild swelling in the entire paw. |
| 3 | The entire paw is swollen. |
| 4 | The mouse cannot grasp the top of a cage's wires due to severe swelling in the entire paw and all toes, or stiff ankles and toes. |

### Experimental Example 5: Confirmation of peptide efficacy on dry eye syndrome

The present inventors observed the efficacy on dry eye syndome using the immunomodulatory peptide candidates prepared according to one example of the present invention. Specifically, to confirm whether the peptides improved dry eye syndrome, changes in dry eye symptoms such as corneal damage and corneal opacity were measured in a benzalkonium chloride (BAC)-induced dry eye disease (DED) model.

### 1) Method of inducing BAC-induced dry eye syndrome model and administering peptide

The present inventors treated BAC as a dry eye syndrome-inducing agent in approximately 12 to 16-week-old C57BL/6 mice. BAC was administered topically by instillation into the right eye twice a day for a total of 16 days. For 4 days (a total of 8 times), only BAC was administered, and starting from day 5, peptides were also administered topically by instillation in the same manner 30 minutes after BAC administration.

### 2) Method of evaluating dry eye syndrome

During the experimental period, visual changes were observed every day, and corneal damage was confirmed under a microscope using 0.2% fluorescein, which is a fluorescent indicator, on days 4, 8, 11, and 16 after the start of administration. Since the area stained with fluorescein indicates corneal damage, the extent of corneal damage was evaluated by calculating the stained area. At the same time, the eyes were observed under a microscope to score the degree of corneal opacity.

### 3) Experimental results

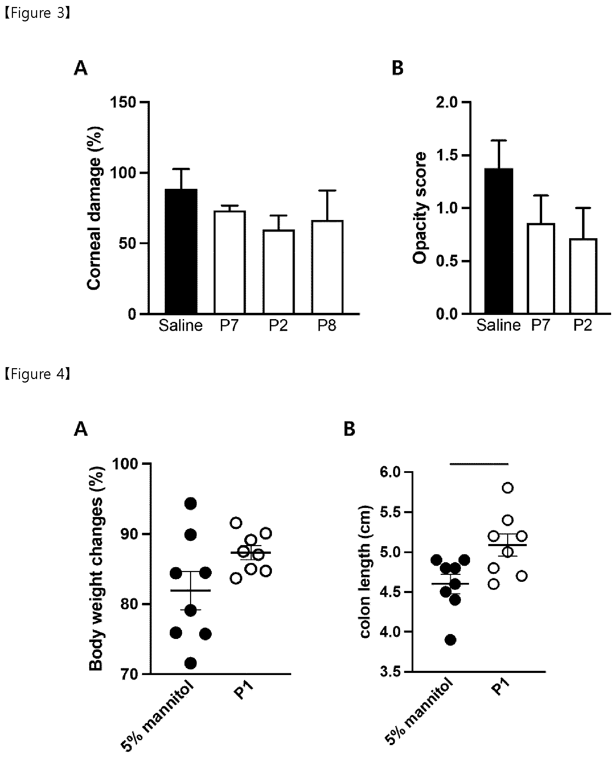

As a result, it was confirmed that corneal damage and corneal opacity caused by BAC were improved upon peptide administration. This suggests that the peptide according to the present invention can improve the symptoms of dry eye syndrome in an individual. The results of analyzing the degree of corneal damage and corneal opacity of the peptide are shown in FIG. 3.

### Experimental Example 6: Confirmation of peptide efficacy on colitis

The present inventors observed the efficacy on colitis using the immunomodulatory peptide candidates prepared according to one example of the present invention. Specifically, to confirm whether the peptides reduce inflammatory responses occurring in the large intestine, changes in weight and intestinal length of an individual were measured in a dextran sulfate sodium (DSS)-induced colitis (inflammatory bowel disease (IBD)) model.

### 1) Method of inducing DSS-induced colitis model and administering peptide

The present inventors used approximately 7 to 8-week-old C57BL/6 mice, and 2.5% DSS as a substance for inducing colitis was dissolved in water and provided ad libitum. After 3 days, the existing water was discarded and newly prepared 2.5% DSS water was available to drink for two more days. After a total of 5 days, it was replaced with fresh water. From the day after providing 2.5% DSS, 100 µL of the peptide was administered intraperitoneally once a day.

### 2) Colitis evaluation method

Body weights were measured every day during the experiment, and after 7 days, the mice were euthanized with CO₂ to measure the intestinal length.

### 3) Experimental results

As a result, it was confirmed that body weight loss was improved upon peptide administration, and the intestinal length shortened by inflammation recovered. This suggests that the peptide according to the present invention can improve colitis symptoms in an individual. The measurement results of the weight and intestinal length of the colitis mice are shown in FIG. 4.

### Experimental Example 7: Confirmation of peptide efficacy on neuroinflammation

The present inventors observed the efficacy on neuroinflammation using the immunomodulatory peptide candidates prepared according to one example of the present invention. Specifically, to determine whether the peptides reduce inflammatory responses in neuronal cells, changes in cytokine gene expression in neuronal cells were measured upon inflammation.

### 1) Preparation of cells and peptides

The present inventors used an SK-N-MC cell line, that is, human neuronal cells, and dorsal root ganglion (DRG) neurons, that is, rat neuronal cells. In this experiment, the cells were cultured in a neuronal cell culture medium under conditions of 37 °C and 5% CO₂ condition. Lipopolysaccharide (LPS; Sigma-Aldrich, #L2630), which is the main component of a gram-negative bacterial cell wall, was used as a substance causing an inflammatory response. A peptide that had been dissolved in water at 1 mM was prepared.

### 2) Experimental method

The cells were plated in a 24-well plate (Costar, #3603) at a density of 2.5 × 10⁵ cells/well. After 24 hours, the prepared cells were treated with the peptide at 1µM. After incubation at 37 °C for 30 minutes, to induce inflammation, 500 ng/mL of LPS was treated. After incubation at 37 °C for 6 hours, the cells in a medium-free state were treated with TRIzol^{™} (Invitrogen, #15596018) to lyse the cells. Through the given experimental method, RNA was obtained, cDNA was produced using a reverse transcriptase (Promega, #M1705). Afterward, a cytokine expression level was measured using SYBR Green (Enzynomics, RT530) and mouse primers of each inflammatory cytokine gene (IL-1β, IL-6).

### 3) Experimental results

As a result, it was confirmed that, in most peptides, the inflammatory cytokines (IL-1β and IL-6) are reduced compared to the control. This shows that the peptide according to the present invention can reduce the neuroinflammatory response in an individual. The results of analyzing the changes in cytokine gene expression according to the peptide treatment are shown in Table 5.

### Experimental Example 8: Confirmation of peptide efficacy on neuroinflammation-induced degenerative brain diseases

The present inventors observed the efficacy on brain diseases involving neuroinflammation using the immunomodulatory peptide candidates prepared according to one example of the present invention. Specifically, to confirm whether the peptides reduce Parkinson's disease based on the inflammatory response inhibition efficacy in neuronal cells, in an MPTP-induced Parkinson's disease animal model, changes in the number of dopaminergic neurons and density of terminal fibers in brain tissue were analyzed.

### 1) Method of inducing MPTP-induced Parkinson's disease model and administering peptide

The present inventors used approximately 7 to 8-week-old C57BL/6 mice, and used 1-methyl-4-phenyl-1,2,3,6-tetrahydropyridine (MPTP) hydrochloride, which induces neuroinflammation, as a brain lesion-inducing substance. 15 mg/kg MPTP was intraperitoneally administered four times to induce the disease, and then selected peptide candidate was intraperitoneally administered a total of seven times once a day for 7 days.

### 2) Method of evaluating Parkinson's disease improvement

The day after the final administration, the mice were anesthetized and perfused with phosphate buffered saline and a fixative (4% paraformaldehyde) to remove blood and fix tissue, and then the brain was extracted. Using the extracted brain, cryoblock was manufactured, and then section slides were crated out of the cryoblock. In the section slides, dopaminergic neurons were stained using TH staining. Afterward, the stained tissue was photographed under a microscope and the recovery of the number of dopaminergic neurons and the density of nerve terminal fibers was analyzed.

### 3) Experimental results

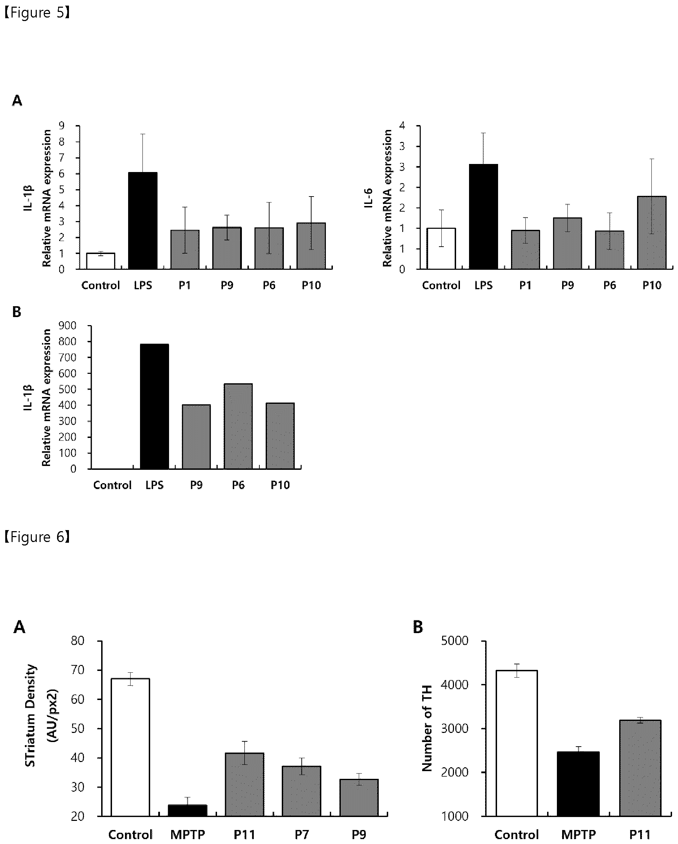

As a result, it was confirmed that the density of nerve terminal fibers of dopaminergic neurons, reduced by MPTP, was restored when the immunomodulatory peptides were administered. This suggests that the peptide according to the present invention can improve Parkinson's disease by recovering dopaminergic neurons affected by neuroinflammation at the individual level through the resolution of inflammation. The results of analyzing changes in the severity score of the peptide are shown in FIG. 6.

### Experimental Example 9: Confirmation of tumor growth inhibition ability by peptide

The present inventors observed tumor growth inhibition ability using the immunomodulatory peptide candidates prepared according to one example of the present invention. Specifically, to confirm whether the peptides inhibit tumor growth, changes in the tumor size were measured in a cancer cell implantation animal model.

### 1) Cancer cell implantation model and peptide administration method

The present inventors used approximately 8-week-old C57BL/6 mice and a CT-26 cell line as colon cancer cells. 2.5×10⁵ cells were subcutaneously implanted at the flank of each mouse to induce tumor growth for about 2 weeks. Animals in which the tumors had grown to a uniform size were selected and randomly assigned to drug administration groups. Afterward, the peptide was intraperitoneally injected five times a week, and 5-fluorouracil (5-FU), which is a positive control drug, was injected twice a week.

### 2) Method of evaluating tumor growth inhibition

During the experiment, body weights and visual changes were observed to confirm the overall health conditions of the animals. Every 3 to 4 days, the length and width of the tumor were measured, which was assume to be an ellipsoid, and the tumor volume as an ellipsoid was calculated. Changes in tumor volume were tracked during the experiment to evaluate tumor growth inhibition efficacy. After the final administration of the substance was completed, blood was collected for analysis of inflammatory factors or tumor-related factors, and a paraffin block was manufactured from the extracted tumor tissue and subjected to tissue analysis using hematoxylin and eosin (H&E) staining or immunohistochemistry.

### 3) Experimental results

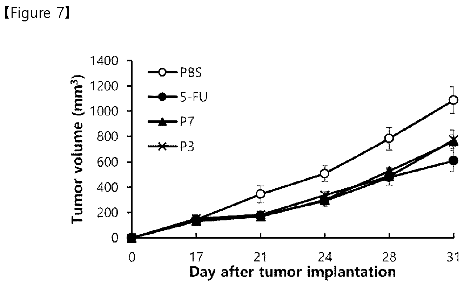

As a result, it was confirmed that the growth of tumors formed by the implantation of the CT-26 cell line was inhibited when the peptide was administered. This suggests that the peptide according to the present invention can inhibit tumor growth through immunomodulation based on the function of inflammation resolution. The results of analyzing changes in severity score of the peptide are shown in FIG. 7.

The present invention has been described with reference to the above-described examples and experimental examples, but this is merely illustrative, and it should be understood by those of ordinary skill in the art that various modifications and equivalent examples and experimental examples can be made therefrom. Therefore, the genuine scope of the present invention should be determined from the technical spirit of the accompanying claims.

## Claims

1. A peptide consisting of an amino acid sequence represented by X1-X2-X3-X4-X5-X6-m,
wherein X1 is absent, or arginine (R), lysine (K), histidine (H), or proline (P);
X2 is methionine (M) or tryptophan (W);
X3 is a basic amino acid such as arginine (R) or lysine (K);
X4 is tyrosine (Y);
X5 is a basic amino acid such as arginine (R) or lysine (K), or an aromatic amino acid such as tryptophan (W) or tyrosine (Y);
X6 is proline (P), tyrosine (Y), or valine (V); and
m is D-methionine.

2. The peptide of claim 1, wherein X1 is absent, or arginine (R), lysine (K), histidine (H), or proline (P);
X2 is methionine (M) or tryptophan (W);
X3 is a basic amino acid such as arginine (R) or lysine (K);
X4 is tyrosine (Y);
X5 is arginine (R) or lysine (K);
X6 is valine (V), tyrosine (Y), or proline (P); and
m is D-methionine.

3. The peptide of claim 1, wherein X1 is absent, or a basic amino acid such as arginine (R) or lysine (K);
X2 is methionine (M) or tryptophan (W);
X3 is a basic amino acid such as arginine (R) or lysine (K);
X4 is tyrosine (Y);
X5 is an aromatic amino acid such as tryptophan (W);
X6 is valine (V), tyrosine (Y), or proline (P); and
m is D-methionine.

4. The peptide of claim 1, wherein X1 is absent, or a basic amino acid such as arginine (R) or lysine (K);
X2 is methionine (M) or tryptophan (W);
X3 is a basic amino acid such as arginine (R);
X4 is tyrosine (Y);
X5is tyrosine (Y);
X6 is valine (V), tyrosine (Y), or proline (P); and
m is D-methionine.

5. The peptide of claim 1, wherein any one selected from the group consisting of an acetyl group, a butanoyl group, a hexanoyl group, and an octanoyl group is added to the N-terminus of the peptide.

6. The peptide of claim 1, wherein the C-terminus of the peptide is amidated.

7. The peptide of claim 1, which consists of any one of the amino acid sequences of SEQ ID NOs: 1 to 127.

8. A peptide consisting of an amino acid sequence represented by X1-X2-X3-X4-X5-X6-m,
wherein X1 is a basic amino acid such as arginine (R) or lysine (K);
X2 is an aromatic amino acid such as tryptophan (W);
X3 is a basic amino acid such as arginine (R) or lysine (K);
X4 is tyrosine (Y);
X5 is arginine (R), lysine (K), leucine (L), asparagine (N), phenylalanine (F), or threonine (T);
X6 is isoleucine (I), threonine (T), tyrosine (Y), or valine (V); and
m is D-methionine.

9. A pharmaceutical composition for preventing or treating an immune disease or cancer, comprising the peptide of any one of claims 1 to 8 as an active ingredient.

10. The pharmaceutical composition of claim 9, wherein the immune disease is atopic dermatitis, psoriasis, infantile eczema, sepsis, conjunctivitis, keratitis, dry eye syndrome, asthma, lung injury, lung inflammation, rheumatoid arthritis, ankylosing spondylitis, ulcerative colitis, Crohn's disease, peritonitis, systemic sclerosis, neuroinflammation, Parkinson's disease, or Alzheimer's disease.

11. An anti-inflammatory cosmetic composition comprising the peptide of any one of claims 1 to 8 as an active ingredient.
